# EUROPEAN PATENT SPECIFICATION

(11) **EP 3 001 818 B1**
(45) Date of publication and mention of the grant of the patent: **20.06.2018**
(21) Application number: 13888727.8
(22) Date of filing: 03.07.2013
(51) Int. Cl.: A61M 16/06, A61M 15/00, A61M 11/02, A61M 21/02, A61M 21/00

(54) **DEVICES AND SYSTEMS FOR FACILITATING FACEMASK COMPLIANCE**
VORRICHTUNGEN UND SYSTEME ZUR ERLEICHTERUNG DER NACHGIEBIGKEIT EINER GESICHTSMASKE
DISPOSITIFS ET SYSTÈMES POUR FACILITER UNE CONFORMITÉ DE MASQUE DE PROTECTION

(43) Date of publication of application: 06.04.2016
(73) Proprietor: Astartein, LLC, Chapel Hill NC 27517-9406 (US)
(72) Inventor: HICKEY Anthony J., Chapel Hill, NC 27517 (US); CROWDER, Timothy M., Durham, NC 27707 (US)
(74) Representative: Yeadon IP Limited
(86) International application number: PCT/US2013/049268
(87) International publication number: WO 2015/002652

(56) References cited:
- EP-A1- 0 661 071
- WO-A1-01/43804
- WO-A1-01/43804
- WO-A1-99/30760
- WO-A1-99/30760
- WO-A2-2010/045408
- WO-A2-2010/045408
- US-A1- 2003 192 547
- US-A1- 2003 192 547
- US-A1- 2005 263 150
- US-A1- 2005 263 150

## Description

### BACKGROUND

Treatment of disease with inhaled aerosols in young children can be difficult. The time required for nebulized aerosols to deliver an effective dose, the "mist" generated and/or the noise of the nebulizer pump can distract and/or frighten a child and result in the child removing the facemask. Boredom and discomfort are additional reasons that a child may remove the facemask. For air jet nebulizers, this often results in wasted drug, which is vented to atmosphere. In addition to waste, efficacy and delivery efficiency are negatively affected. Further, there may be negative facial and/or ocular effects due to the drug being deposited outside the facemask.

In addition, the transition from nebulized drugs to those delivered by a metered dose inhaler (MDI) with a spacer and facemask can be difficult due to the newness of the experience and the difference in sensation of inhaling propellant-based aerosols. As a result, children may remove the facemask leading to the same drawbacks described above.

EP 0 661 071 A1 describes a device for continuous positive airway pressure (CPAP) treatment. The starting of administration of CPAP treatment occurs when it is determined that the patient is wearing the mask. The ceasing of the administration of CPAP treatment occurs when it is determined that the patient is no longer wearing the mask. The mask comprises a pair of conductive plates, a pair of capacitive plates or an inductive coiled wire.

### SUMMARY

Some embodiments of the present invention are directed to a facemask. The invention is defined by the appended claim 1. The facemask includes a facemask body having a first end including an opening configured to receive at least a portion of a user's face and a skin contact perimeter extending along a periphery of the opening. The skin contact perimeter is configured to sealingly engage with a user's face. The facemask includes a plurality of electrical contacts, with each electrical contact disposed at a respective region of the skin contact perimeter. Each electrical contact is configured to sense when the respective region of the skin contact perimeter is sealingly engaged with a user's face. A control circuit is disposed on the facemask body. The control circuit is configured to receive a signal from each electrical contact when the respective region of the skin contact perimeter is sealingly engaged with a user's face. The control circuit is configured to initiate output of an audible stimulus and/or a visual stimulus in response to receiving a concurrent signal from all of the plurality of electrical contacts.

The facemask body may include a port configured to receive medicament from a medicament delivery device, wherein the opening and the port are in fluid communication such that medicament received in the port can be delivered to the opening. The port may be configured to operatively connect with at least one of a nebulizer and a metered dose inhaler (MDI) spacer. The facemask body may define a cavity, with at least a portion of the cavity extending between the opening and the port.

In some embodiments, the facemask includes a speaker disposed on the facemask body, and the control circuit is configured to control the speaker to output an audible stimulus. The control circuit may be configured to control the speaker to output a children's melody and/or song as the audible stimulus.

In some embodiments, the facemask includes at least one light emitting diode (LED) disposed on the facemask body, with the control circuit configured to illuminate the at least one LED as a visual stimulus. The facemask may include a plurality of LEDs, with the control circuit configured to illuminate the LEDs in a sequence as the visual stimulus. In some embodiments, the facemask includes a speaker disposed on the facemask body and a plurality of LEDs disposed on the facemask body, and the control circuit is configured to control the speaker to output an audible stimulus and illuminate the LEDs as a visual stimulus.

The control circuit is configured to stop the audible stimulus and/or visual stimulus when one of the electrical contacts does not sense that the respective portion of the skin contact perimeter is sealingly engaged with a user's face.

The facemask may include a power source disposed on the facemask body. Each electrical contact may be a switch configured to be actuated when the respective region of the skin contact perimeter is sealingly engaged with a user's face, and the switches may be configured to electrically connect the power source and the control circuit such that, when all the switches are concurrently actuated, the power source is electrically connected with the control circuit to initiate output of the audible stimulus and/or the visual stimulus. In some embodiments, at least three switches are spaced-apart along the skin contact perimeter. The switches may be positioned such that, when the facemask is sealingly engaged with a user's face, one of the switches is actuated by the bridge of the user's nose and two of the switches actuated by the user's face on opposite sides of the user's mouth. In some other embodiments, each electrical contact is a capacitive sensor configured to detect contact with skin.

The facemask may include a timer configured to measure an amount of time that a concurrent signal is received from all the plurality of electrical contacts. The timer may be configured to initiate output of an alarm signal when the measured amount of time reaches a threshold value and/or does not reach a threshold value. The facemask may include a memory configured to store data received from the timer.

In some embodiments, the facemask includes a transmitter disposed on the facemask body, with the transmitter being configured to transmit a wireless signal to an outside device that is configured to output the audible stimulus and/or the visual stimulus. In some embodiments, the facemask includes a transmitter disposed on the facemask body, with the transmitter configured to transmit a wireless signal to an outside device when a concurrent signal is received from all the plurality of electrical contacts such that the outside device can display and/or record data based on the wireless signal. In some embodiments, the facemask is configured to be wiredly connected to an outside device that is configured to output the audible stimulus and/or the visual stimulus.

The facemask may be used in combination with a nebulizer assembly including a chamber configured to hold liquid medicament and a pump in fluid communication with the chamber. The pump is configured to supply pressurized fluid to the chamber to form an aerosol, and the chamber is in fluid communication with the port of the facemask body.

The facemask may be used in combination with an MDI spacer having a body including first and second opposite end portions, with the first end portion of the spacer adapted to receive an MDI, and with the second end portion operatively connected to the port of the facemask body. A plurality of LEDs may be disposed on the spacer body, with the control circuit configured to illuminate the LEDs as the visual stimulus. At least one light string may be disposed around a circumference of the spacer body and/or along a length of the spacer body, with the control circuit configured to illuminate the at least one light string as the visual stimulus.

Some embodiments of the present disclosure are directed to methods for improving juvenile compliance with inhaled medication administration. The method includes providing a facemask. The facemask includes a body having an opening at an end thereof with a skin contact perimeter arranged around the opening and at least three switches in a spaced-apart relationship at the skin contact perimeter. The facemask also includes at least one output device in electrical communication with the switches and configured to output an audible stimulus and/or a visual stimulus. The method includes receiving at least a portion of a child's face in the opening. The method includes outputting an audible stimulus and/or a visual stimulus from the at least one output device in response to concurrent actuation of all switches indicating that the skin contact perimeter is sealingly engaged with the child's face. The method includes halting the audible stimulus and/or the visual stimulus from the at least one output device when at least one of the switches is not actuated indicating that at least a portion of the skin contact perimeter is not sealingly engaged with the child's face.

It is noted that any one or more aspects or features described with respect to one embodiment may be incorporated in a different embodiment although not specifically described relative thereto. That is, all embodiments and/or features of any embodiment can be combined in any way and/or combination. Applicant reserves the right to change any originally filed claim or file any new claim accordingly, including the right to be able to amend any originally filed claim to depend from and/or incorporate any feature of any other claim although not originally claimed in that manner. These and other objects and/or aspects of the present invention are explained in detail in the specification set forth below.

Further features, advantages and details of the present invention will be appreciated by those of ordinary skill in the art from a reading of the figures and the detailed description of the preferred embodiments that follow, such description being merely illustrative of the present invention.

### BRIEF DESCRIPTION OF THE FIGURES

**Figure 1** is a perspective view of a facemask according to some embodiments of the invention.
**Figure 2** is a block diagram illustrating components used in facemasks according to some embodiments of the invention.
**Figure 3** is a perspective view of a facemask according to some other embodiments of the invention.
**Figure 4** is a perspective view of a facemask connected with a nebulizer according to some embodiments of the invention.
**Figure 5** is a perspective view of a facemask connected to a spacer and a metered dose inhaler (MDI) according to some embodiments of the invention.
**Figure 6** is a perspective view of an exemplary facemask connected to an MDI spacer according to some embodiments of the invention.
**Figure 7A** is a perspective view of a facemask connected to an alternative MDI spacer according to some other embodiments of the invention.
**Figure 7B** is a partial perspective view of the facemask of **Figure 7A** used with a further alternative MDI spacer according to some embodiments of the invention.
**Figure 8** is a perspective view of an exemplary facemask configured to wirelessly communicate with one or more devices according to some embodiments of the invention.
**Figure 9A** is a schematic illustration showing exemplary contact points of a facemask for a respective patient's face according to embodiments of the invention.
**Figure 9B** is a schematic illustration showing the facemask of **Figure 9A** sealingly engaged with the patient's face.
**Figure 9C** is a schematic illustration showing the facemask of **Figure 9A** that is not sealingly engaged with the patient's face and which can be electrically detected due to insufficient electrical contact according to embodiments of the invention.

### DETAILED DESCRIPTION OF EMBODIMENTS OF THE INVENTION

The present invention now will be described more fully hereinafter with reference to the accompanying drawings, in which illustrative embodiments of the invention are shown. In the drawings, the relative sizes of regions or features may be exaggerated for clarity. This invention may, however, be embodied in many different forms and should not be construed as limited to the embodiments set forth herein; rather, these embodiments are provided so that this disclosure will be thorough and complete, and will fully convey the scope of the invention to those skilled in the art.

It will be understood that when an element is referred to as being "coupled" or "connected" to another element, it can be directly coupled or connected to the other element or intervening elements may also be present. In contrast, when an element is referred to as being "directly coupled" or "directly connected" to another element, there are no intervening elements present. Like numbers refer to like elements throughout.

In addition, spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the expression "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

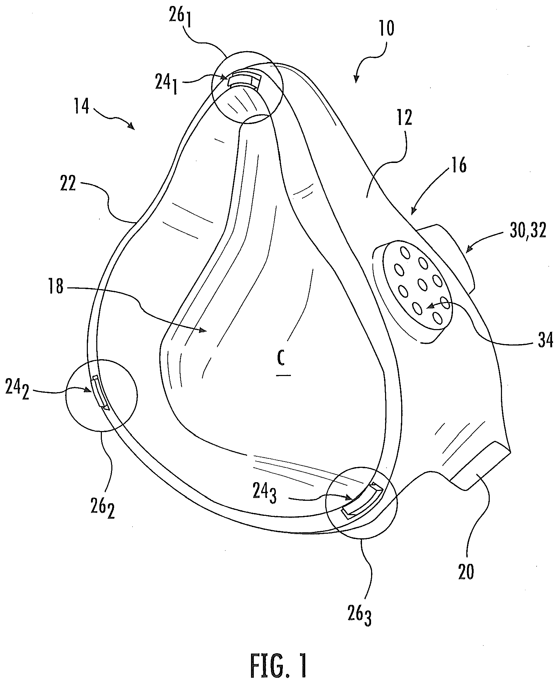

Generally stated, embodiments of the invention provide facemasks that provide audio or visual outputs when the facemask is sealingly engaged with a patient's face. A facemask **10** according to some embodiments of the invention is shown in **Figure 1****.** The facemask **10** includes a body or shell **12** having a first or rear end **14** that contacts a face of a patient and an opposing second or forward end **16.** The body **12** defines a cavity **C.** The body **12,** and therefore the cavity **C,** tapers from the first end **14** to the second end **16.** An opening **18** is defined at the first end **14** of the body **12.** The opening **18** is configured to receive a portion of a patient's or user's face such that the nose is disposed in the cavity **C.**

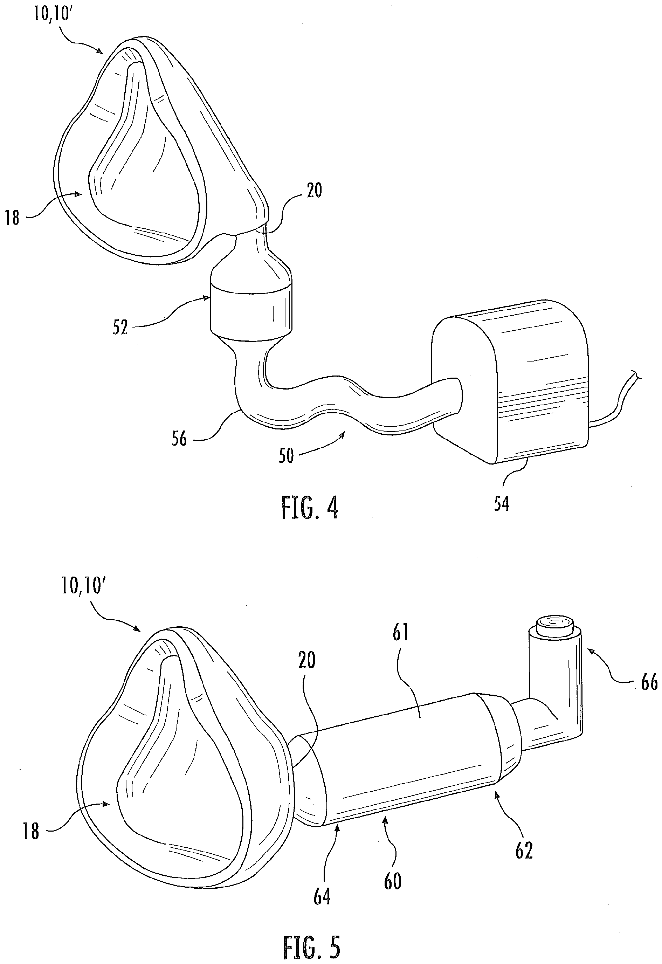

The body **12** includes a port **20** configured to receive medicament from a medicament delivery device. In position, medicament may be received at the port **20,** flow through at least a portion of the cavity **C,** and be inhaled by a patient or user. The port **20** may face in a direction generally perpendicular to the opening **18** (for example, generally downwardly facing as shown in **Figures 1** and **4**), which may be a desired orientation for use with a nebulizer. Alternatively, the port **20** may be positioned at the second end **16** of the facemask body **12** (**Figure 5**), which may be a desired orientation for use with a spacer or a valved holding chamber used in connection with a metered dose inhaler (MDI) or pressurized metered dose inhaler (pMDI). It is contemplated that the port **20** may be positioned on or extend from the facemask body **12** in any orientation and/or position, e.g., at the second end **16** of the body **12** or between the second end **16** of the body **12** and the first end **14** of the body **12.**

Still referring to **Figure 1****,** the facemask **10** includes a skin contact perimeter **22** at the first end **14** of the body **12.** The perimeter **22** is configured to sealingly engage with a patient's or user's face. That is, as the facemask **10** is brought toward the patient's face, a portion of the patient's face is received through the opening **18,** and the skin contact perimeter **22** seals against the patient's face (e.g., seals against the nose, cheek and/or chin regions). The skin contact perimeter **22** extends along a periphery of the opening **18** and is typically curvilinear and may include a triangulated incline to accommodate a nose of a patient. Although not illustrated, the facemask **10** may include a strap which, as known to those of skill in the art, may be configured to loop around a back or rear of a patient's head to help keep the facemask in place during use.

The facemask body **12** may be formed as a single monolithic piece such that the first end **14** of the body **12** defines the skin contact perimeter **22.** The body **12** may be formed of a flexible, resilient material so as to conform to the patient's face and provide a tight seal. In some other embodiments, the skin contact perimeter **22** may be separate contact component or member that is attached to the first end **14** of the body **12.** In this regard, the skin contact perimeter **22** may be formed of a flexible, resilient material and the body **12** may be formed of a somewhat more rigid material. If the skin contact perimeter **22** is a separate contact component or member, it can be integrally or releasably attached.

A plurality of spaced-apart electrical contacts or inputs (e.g., sensors and/or switches) are disposed about the skin contact perimeter **22.** The electrical contacts or inputs may be attached to an outer surface of the skin contact perimeter **22** or may be at least partially embedded in the perimeter **22** and/or the facemask body **12.** Three spaced-apart electrical contacts or inputs **24₁, 24₂, 24₃** are shown in the embodiment illustrated in **Figure 1****.** Each electrical contact or input **24₁, 24₂, 24₃** is disposed at a corresponding edge region **26₁, 26₂, 26₃** of the skin contact perimeter **22.** Each electrical contact or input **24₁, 24₂, 24₃** is configured to sense when its corresponding region **26₁, 26₂, 26₃** of the skin contact perimeter **22** is in contact with the patient's face. When all three electrical contacts or inputs **24₁, 24₂**, **24₃** are in contact, the skin contact perimeter **22** can be identified as in proper sealed engagement.

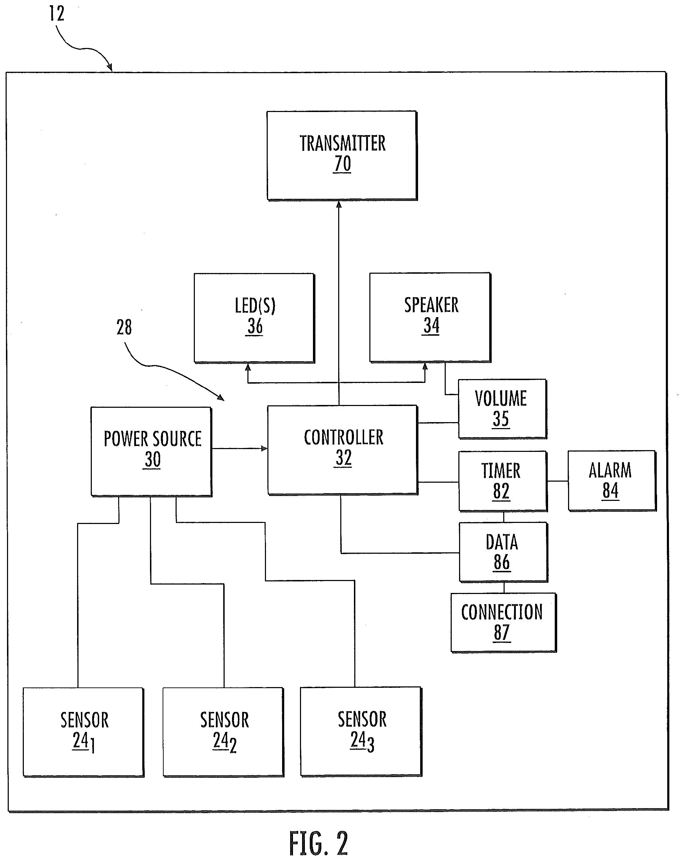

Turning to **Figure 2****,** the facemask **10** may include or be in communication with a control circuit **28.** The control circuit **28** may include a power source **30** (e.g., a battery) and/or a controller **32** (e.g., a microcontroller or a microprocessor). The power source **30** and/or the controller **32** may be disposed on the facemask body **12,** as shown in **Figure 1****.** The power source **30** and/or the controller **32** may be attached to an outer surface of the body **12** or may be at least partially embedded in the body **12** or may be suspended or otherwise attached to the facemask **10** or a strap associated therewith. The control circuit **28** is configured to monitor and/or receive a signal from each of the electrical contacts or inputs **24₁, 24₂**, **24₃** that indicates that the corresponding region **26₁, 26₂, 26₃** of the skin contact perimeter **22** is in contact with skin and thus sealingly engaged with the patient's face.

The control circuit **28** is configured to initiate output of an audible stimulus and/or a visual stimulus in response to receiving a concurrent signal from each of the plurality of electrical contacts or inputs **24₁, 24₂**, **24₃**. For example, in the embodiment shown in **Figure 1****,** the control circuit **28** (or the controller **32**) can be configured to send a signal to a speaker **34** such that the speaker outputs an audible stimulus. As illustrated, the speaker **34** may be disposed on the facemask body **12.** The speaker **34** may be attached to an outer surface of the body **12** or may be at least partially embedded in the body **12** or may be suspended or otherwise attached to the facemask **10** or a strap associated therewith (e.g., the speaker **34** may be attached as an earpiece). Any suitable speaker may be employed; an exemplary suitable speaker is a piezo speaker.

The audible stimulus may be a melody or song that is particularly pleasing, soothing and/or stimulating to a child, and therefore may encourage the child to retain the facemask in a sealing relationship with his or her face. The melody or song may be stored on the controller **32.** In some embodiments, the controller **32** stores a plurality melodies and/or songs. The controller **32** may control the speaker **34** to output different melodies in a predetermined or a random sequence. In some embodiments, the controller **32** and/or the speaker **34** is or includes a melody integrated circuit. In some embodiments, the melodies/songs may be customized and user selectable via a display or other input. For example, the melodies/songs may be downloaded and/or selected using a computer, electronic notepad, smartphone, etc.

The volume of the audible stimulus may be adjustable or controllable, for example using a volume control **35** (**Figure 2**). The volume control **35** may be or include button(s), a dial or the like. The volume control **35** may be disposed on the facemask body **12** or elsewhere as described above in connection with the speaker **34.** The controller **32** may adjust the signal sent to the speaker **34** and/or the output of the speaker **34** in response to adjustment of the volume control **35.** The volume control **35** may be adjusted by the patient, a caregiver and/or supervising staff. For example, it may be desirable for a supervising staff member to adjust the volume to accommodate the wishes of a child patient. Generally speaking, the volume control **35** may be included to allow for adjustment of the volume of the audible stimulus to an appropriate level for the hearing of the patient (e.g., a child patient).

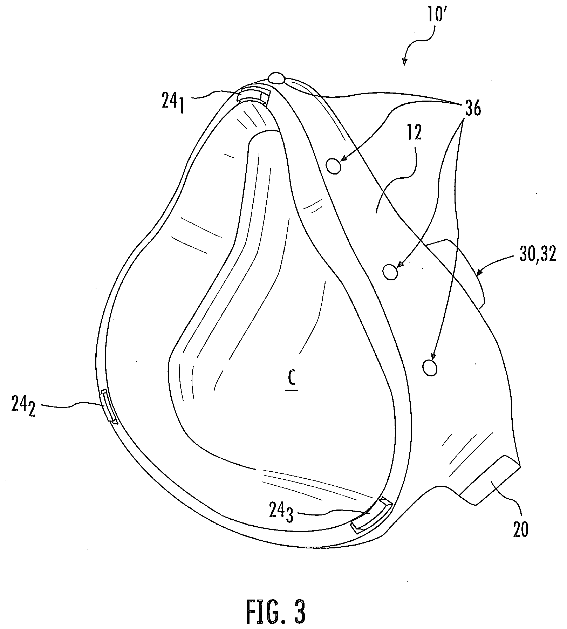

A facemask **10'** according to some embodiments is illustrated in **Figure 3****.** The facemask **10'** includes all the same features of the facemask **10** of **Figure 1** with the speaker **34** being optional. The facemask **10'** includes a plurality of visual indicators **36,** such as light emitting diodes (LEDs), disposed on the facemask body **12.** The LEDs **36** may be attached to an outer surface of the body **12** or may be at least partially embedded in the body **12.** The LEDs **36** may be disposed in a spaced-apart relationship along at least a portion of a periphery of the body **12,** along at least a major portion of the periphery of the body, and along the entire or substantially the entire periphery of the body in various embodiments.

In response to receiving a concurrent signal from each of the plurality of electrical contacts or inputs **24₁, 24₂**, **24₃**, the control circuit **28** (or the controller **32)** is configured to illuminate the LEDs **36** to output a visual stimulus. The controller **32** may be configured to illuminate the LEDs **36** in a particular or predetermined sequence; for example, adjacent LEDs **36** may illuminate sequentially in one direction, giving the effect that the emitted light is "circling" the facemask body **12.** Other predetermined sequences are contemplated; it is also contemplated that the controller **32** may illuminate the LEDs **36** in a random duration and/or order. Additionally or alternatively, the LEDs **36** may be of varying color. The visual stimulus created by the LEDs **36** may be particularly pleasing, soothing and/or stimulating to a child, and therefore may encourage the child to retain the facemask in a sealing relationship with his or her face.

The various features of the facemask **10** (**Figure 1**) and the facemask **10'** (**Figure 3**) may be combined. In particular, the facemask **10** may include the speaker **34** and the LEDs **36** to therefore provide both audible and visual stimuli in response to receiving a concurrent signal from each of the plurality of electrical contacts or inputs **24₁, 24₂**, **24₃**. The audible and visual stimuli may be provided consecutively. For example, the controller **32** may be configured to control the output of the speaker **34** to play a melody and at the same time configured to control the LEDs **36** such that one or more of the LEDs **36** illuminates for each tone of the melody (e.g., a different LED or set of LEDs and/or colors of LEDs for each tone). Although only one controller **32** is illustrated, it is contemplated that more than one controller may be employed (e.g., one to control output of the speaker **34** and one to illuminate the LEDs **36**).

In some embodiments, the electrical contacts or inputs **24₁, 24₂**, **24₃** are switches. Each switch **24₁, 24₂**, **24₃** is configured to be actuated in response to sealing engagement of the corresponding region **26₁, 26₂, 26₃** (**Figure 1**) of the skin contact perimeter **22** and the patient's face. When all switches **24₁, 24₂**, **24₃** are concurrently actuated, the power supply **30** is electrically connected to and supplies power to the controller **32,** which in turn causes the controller to control the output of the speaker **34** and/or the LEDs **36.** In some other embodiments, the electrical contacts or inputs **24₁, 24₂**, **24₃** are contact sensors configured to sense contact with skin (i.e., the patient's face); for example, the sensors **24₁, 24₂**, **24₃** may be capacitive sensors.

The facemasks **10, 10'** may be suitable for use with a nebulizer. Turning to **Figure 4****,** the facemasks **10, 10'** are shown in use with a nebulizer assembly **50.** The nebulizer assembly **50** can include a nebulizer or a nebulizer chamber **52** in which medicine is held. The medicine can be in liquid formulation or other suitable formulation. A compressor or pump **54** is connected to the nebulizer **52** by tubing **56.** The pump **54** causes compressed air or oxygen to flow at high velocity through the medicine to turn it into an aerosol. The nebulizer **52** is in fluid communication with the facemask **10, 10'** via the port **20,** and the aerosol may be inhaled by the patient via the opening **18.** Some embodiments of the present invention are directed to assemblies and systems that include the facemask **10** or **10'** and a nebulizer (e.g., the nebulizer assembly **50**).

Facemasks **10, 10'** according to embodiments of the present invention may be suitable for use with a spacer or a valved holding chamber used in connection with an MDI or a pMDI. A spacer **60** is shown in **Figure 5****.** The spacer **60** has an elongated body **61** with first and second opposed end portions **62, 64.** The first end portion **62** is adapted to receive an MDI or a pMDI **66.** The second end portion **64** is adapted to operatively connect with the facemask **10, 10'** (e.g., via the port **20**). In this regard, MDI **66** is in fluid communication with the facemask **10, 10',** and medicament released from the MDI upon actuation of the MDI may be inhaled by a patient via the first opening **18.**

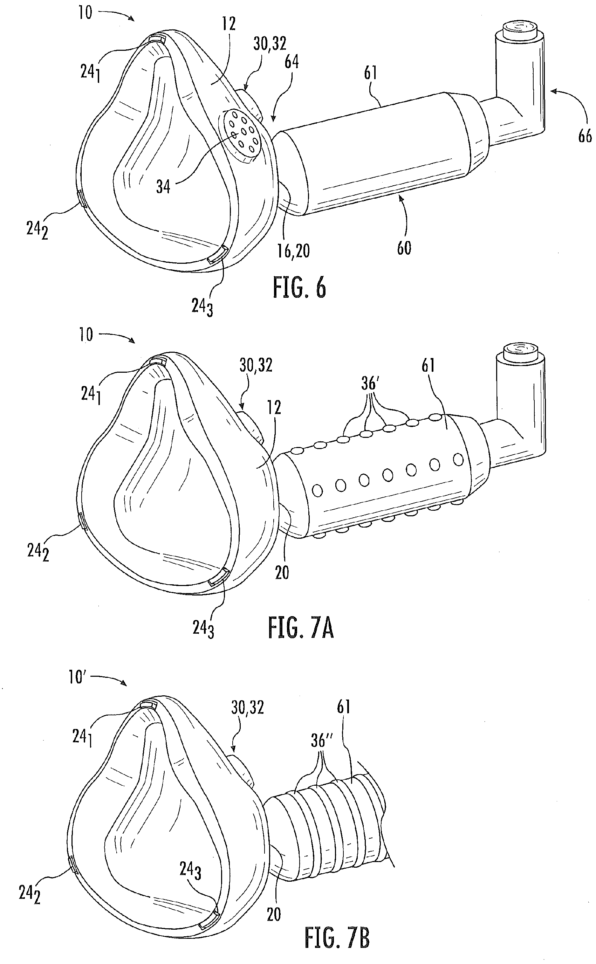

Exemplary facemasks **10, 10'** that may be used with MDI spacers are illustrated in **Figures 6, 7A** and **7B****.** In this embodiment, the port **20** is disposed on the second or forward end **16** of the facemask body **12.** The second end portion **64** of the spacer body **61** may be connected to or operatively connected to the port **20.** This provides an "in-line" orientation of the facemask **10, 10'** and the spacer **60,** which may be desirable with the use of spacers or valved holding chambers.

The facemask **10'** (**Figures 7A** and **7B**) includes the electrical contacts or inputs **24₁, 24₂**, **24₃** but the light emitting units can be disposed on the spacer body 61 as opposed to on the facemask body **12.** As shown in **Figure 7A****,** a plurality of discrete LEDs **36'** may be arranged around a circumference of the spacer body **61** and/or along at least a portion of a length of the spacer body **61.** As shown in **Figure 7B****,** at least one light string **36"** (e.g., LED light string) may be disposed on the spacer body **61** and arranged around a circumference of the spacer body **61.** For example, at least one light string **36"** may be helically disposed along the spacer body **61** or a plurality of light strings **36"** may be annularly disposed around the spacer body **61** in a spaced-apart relationship. It is contemplated that a speaker (e.g., the speaker **34** shown in **Figure 6**) may be employed in connection with the facemask **10'.** The speaker may be disposed on the facemask body **12,** on the spacer body **61** or on some other component, such as an intermediate component between the spacer **60** and the facemask **10',** or may otherwise be attached to or in communication with the facemask **10'.**

The power supply **30** and controller **32** are shown as disposed on the facemask body **12** in **Figures 6, 7A** and **7B****.** It is contemplated that one or both of these components may be disposed on the spacer body **61,** or on an intermediate component between the facemask **100'** and the spacer **60.** In this regard, conductive wires, traces or flex circuit(s) may run from each of the electrical contacts or inputs **24₁, 24₂**, **24₃** to the power supply **30,** for example.

The facemasks **10, 10'** used with spacers can provide similar stimuli to the spacers **10, 10'** used with nebulizers. An advantage of having similar stimuli on both nebulizer and spacer facemasks is that a child may be transitioned from one device to another, which usually occurs around age six.

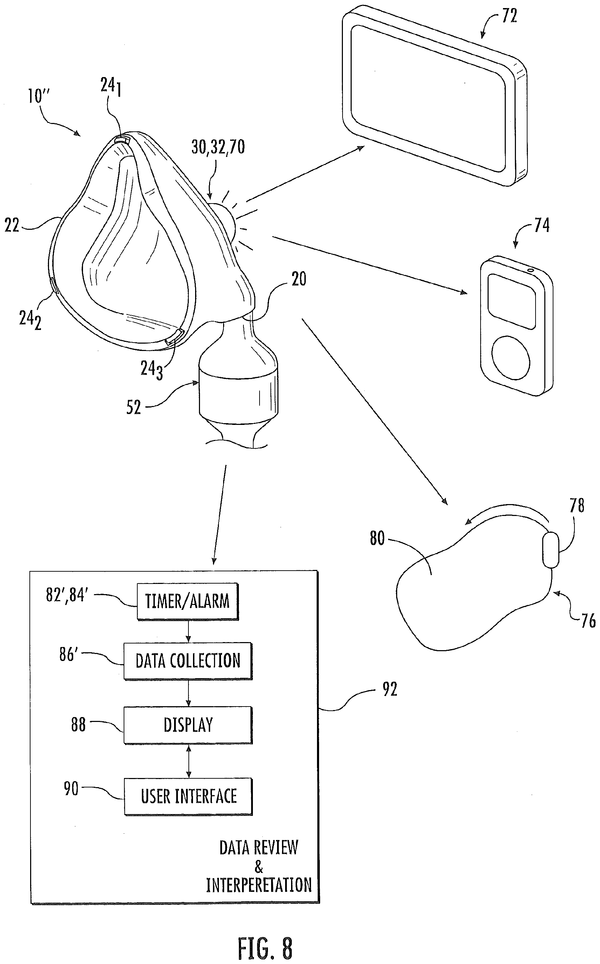

A facemask **10"** according to some other embodiments of the invention is shown in **Figure 8****.** Like the previously described facemasks, the facemask **10"** includes electrical contacts or inputs **24₁, 24₂**, **24₃** to sense whether a patient's face is sealingly engaged with the skin contact perimeter **22** of the facemask **10".** The facemask **10"** includes a wireless transmitter **70.** In response to receiving a concurrent signal from each of the plurality of electrical contacts or inputs **24₁, 24₂**, **24₃**, the power source **30** is electrically connected to the controller **32,** which in turn controls the wireless transmitter **70** to establish a wireless connection with and transmit wireless signals to one or more outside devices that can provide audible and/or visual stimulus. The wireless connection may be any suitable wireless connection such as a radio frequency (RF) connection, a Bluetooth® connection, a wireless local area network connection (e.g., 802.11) and the like.

The transmitter **70** may establish a wireless connection with and transmit wireless signals to a display device **72** that is configured to receive wireless signals and, in response, output visual stimulus and, optionally, audible stimulus. For example, the display device **72** may be a monitor, television, smartphone or a tablet computer. The display device **72** may be configured to play children's movies, children's shows and the like.

The transmitter **70** may also establish a wireless connection with and transmit wireless signals to an audio or music storage device **74** that is configured to receive wireless signals and, in response, to output audible stimulus. The device **74** may be an MP3 player, an iPod®, a smartphone and the like. The device **74** may be loaded with melodies, songs, children's stories and so forth. At least one of the devices **72, 74** may be a smartphone, tablet computer or the like configured to run applications that may provide the audible and/or visual stimuli and that may monitor and/or store data associated with facemask compliance.

The transmitter **70** may also establish a wireless connection with and transmit wireless signals to a toy **76** that is configured to receive wireless signals and, in response, to activate thereby providing visual and/or audible stimulus. As just one example, the toy may be a train **78** that is configured to advance along a track **80** in response to receiving wireless signals from the transmitter **70.**

Although not illustrated, it is contemplated that the facemasks **10, 10', 10"** described above may be configured to receive a wire or cable so as to establish a wired connection to an outside device, such as the devices **72, 74, 76** shown in **Figure 8****.** As described above, the devices **72, 74, 76** can output audible and/or visual stimuli when the facemask is sealingly engaged with a patient's face.

The devices **72, 74, 76** may serve to hold a child's attention an extended period of time to facilitate proper positioning of the facemask during the entire treatment. This may be useful with nebulizer therapy, for example, which often requires 15-30 minutes to deliver a dose of therapeutic aerosol.

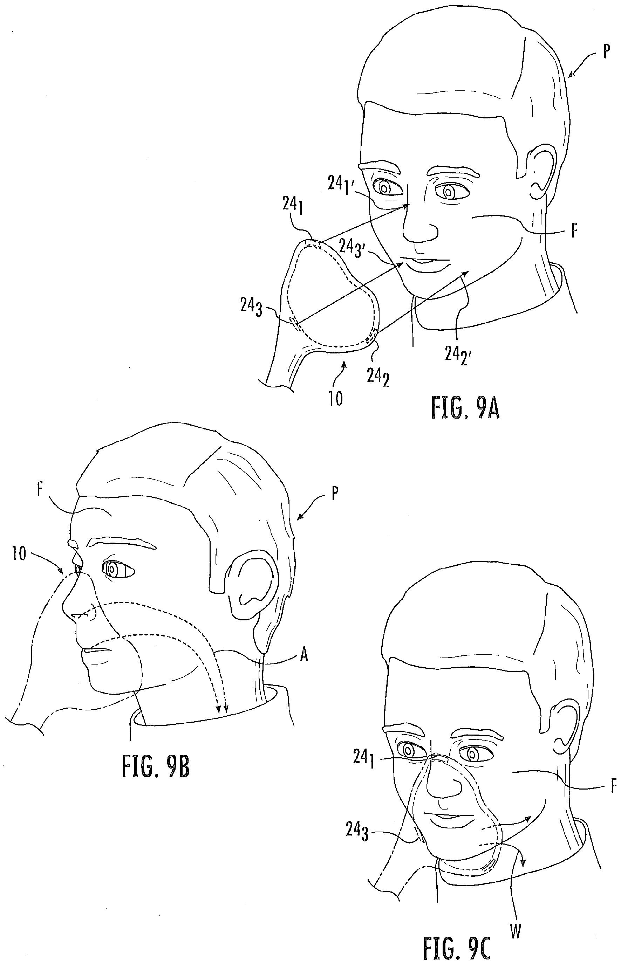

As discussed above, at least three spaced-apart switches **24₁, 24₂**, **24₃** may be employed on the facemasks **10,10', 10"** to enhance compliance. Multiple points of contact are desirable in order to accurately determine that a proper seal has been established so that aerosol may be effectively delivered to the patient. As shown in **Figure 9A****,** as the facemask **10** is brought toward a face **F** of a patient **P,** each one of the switches **24₁, 24₂**, **24₃** contact the patient's face F at a respective point **24₁', 24₂', 24₃'**. For example, and as illustrated, the contact point **24₁'** may be on the bridge of the patient's nose and the contact points **24₂', 24₃'** may be on opposite sides of the patient's mouth (e.g., on opposite cheeks). Therefore, in some embodiments, when the facemask is sealingly engaged with a patient's face, one of the switches **24₁** is actuated by the bridge of the patient's nose and two of the switches **24₂**, **24₃** are actuated by the patient's face on opposite sides of the patient's mouth. **Figure 9B** shows the facemask **10** sealingly engaged with the patient's face **F.** With the facemask **10** sealingly engaged with the patient's face **F,** the aerosol medication (shown as **A**) is effectively inhaled by the patient **P** with little to no waste.

On the other hand, and as shown in **Figure 9C****,** if the facemask is not properly sealed with the patient's face, there will be aerosol waste, shown as **W.** **Figure 9C** illustrates that the positioning and functionality of the switches **24₁, 24₂**, **24₃** may advantageously determine when a suitable seal has been established. As shown in **Figure 9C****,** only the switches **24₁, 24₃** are in contact with skin, and therefore a lack of seal near where switch **24₂** would have been positioned is not detectable. It is contemplated that as few as two switches may be employed, for example the switches **24₂**, **24₃**, as a facemask may be configured to conform around and above the patient's nose. It is also contemplated that more than three switches may be provided, for example 4, 5, 6, 7, 8, 9, 10 or more switches. It is believed that three switches establish a suitable number of contacts to accurately detect whether the facemask is sealingly engaged with the patient's face.

Referring back to **Figure 2****,** a timer **82** and/or an alarm **84** may be employed. The timer **82** may be electrically connected with the electrical contacts **24₁, 24₂**, **24₃** and/or the control circuit **28** (e.g., the power source **30** and/or the controller **32**). The timer **82** may be configured to measure or an amount of time or a duration that the facemask **10, 10', 10"** is sealingly engaged with the patient's face; that is, the amount of time a concurrent signal is received from each of the plurality of electrical contacts **24₁, 24₂**, **24₃**. In some embodiments, the timer **82** is integrated with the controller **32.** In some embodiments, the controller **32** is configured to measure the amount of time or duration that the facemask is sealingly engaged with the patient's face and the timer **82** may be omitted.

The alarm **84** may receive an alarm signal from the timer **82** and/or the controller **32.** The alarm **84** may be configured to receive the alarm signal and provide an audible and/or visual alert when a certain event has occurred. For example, the alarm **84** may output an audible and/or visual alert when the amount of time or duration a concurrent signal received from the electrical contacts **24₁, 24₂**, **24₃** (indicating that the facemask **10, 10', 10"** is sealingly engaged with the patient's face) reaches or exceeds a predetermined threshold value. The threshold value may be known by or stored in the timer **82** and/or the controller **32** and, in some embodiments, may be user selectable. In this regard, the alarm **84** may provide an alert when the appropriate amount of medication has been delivered to the patient. The alarm **84** may also output an audible and/or visual alert when the amount of time or duration a concurrent signal received from the electrical contacts **24₁, 24₂**, **24₃** does not reach the threshold value. In this situation, the alarm **84** may provide an alert that the facemask **10, 10', 10"** has become disengaged and that attention should be given to the patient. In some embodiments, the speaker **34** and/or the LEDs **36, 36', 36"** may be configured to output the audible and/or visual alerts and a separate alarm component may not be needed.

The controller **32,** the timer **82** and/or the alarm **84** may communicate with a data collection system or a memory **86** that is configured to collect data associated with the use of the facemask **10, 10', 10".** The data collection system **86** may collect data such that duration and continuity of medication delivery may be monitored (e.g., by medical staff or some other supervisor). The data may indicate the length of time the facemask was sealingly engaged with the patient's face and may indicate any "gaps" during treatment (e.g., removal or disengagement of the facemask) so that compliance may be monitored and/or reviewed. A connection or port **87** may be provided for connection of a device to retrieve data from the data collection system **86.** As one example, the connection **87** may be a USB port configured to receive a memory stick or thumb drive, which may then be connected to a personal computer or other device such that the retrieved data may be reviewed and analyzed. As another example, the connection **87** may allow direct connection to a device such as a tablet computer, smartphone or the like. The data may be provided to the device dynamically such that the data may be viewed in real time. It may also be stored on the device for later review.

Where used, the timer **82,** the alarm **84,** the data collection system **86** and/or the connection **87** may be disposed on the facemask body **12** or elsewhere as described above in connection with the power source **30** and the controller **32.** Thus, the continuity and/or duration of medication delivery and data associated therewith may be monitored at or near the facemask **10, 10', 10".**

In some embodiments, and as shown in **Figure 8****,** the facemask **10"** may wirelessly communicate with a device **92.** The device **92** may include a timer **82',** an alarm **84',** a data collection system or memory **86',** a display **88** and/or a user interface **90.** For example, in response to receiving a concurrent signal from each of the plurality of contacts **24₁, 24₂**, **24₃**, the wireless transmitter **70** may establish a connection and transmit a wireless signal to the device **92,** and the timer **82'** and/or the alarm **84'** may provide the functionality of the timer **82** and/or the alarm **84** as described above. The data collection system **86'** may collect data associated with the use of the facemask **10"** in the same manner described above in connection with the data collection system **86.** The display **88** may be used to display the data from the data collection system **86'** in real time; alternatively, a user may review and interpret data on the display **88** at a later time. A user may use the user interface **90** to interpret, manipulate and/or review the data on the display **88.** The user interface **90** may be integrated with the display **88** (e.g., a touch display).

The device **92** may be a personal computer, tablet computer, smartphone or the like to generally monitor and store data associated with facemask compliance. It is contemplated that one or more of the timer **82',** the alarm **84'** and/or the data collection system **86'** may be omitted or its equivalent may be provided at or near the facemask **10"** as described above. For example, the data collection system **86** may be provided at or near the facemask **10"** and data from the data collection system **86** may be wirelessly transmitted by the transmitter **70** to the device **92.**

The facemasks described herein can improve juvenile patient compliance with inhaled medication administration. The contact switches help ensure that the audible stimulus and/or visual stimulus are provided only when a proper seal has been established and is maintained. The audible stimulus and/or visual stimulus can help hold a child's attention during dosing. The child may become discouraged when the audible stimulus and/or visual stimulus is halted due to the child removing the mask, and therefore the child may learn to retain the mask in place during treatment. The facemasks may enhance the experience for the child by employing the audible stimulus and/or visual stimulus (e.g., distractions) to encourage continuous placement of the mask, and thereby improve patient compliance and effective delivery of the drug.

The multiple contact switches providing multiple points of contact help establish that a proper seal is established and maintained. Properly sealed contact with the face not only helps with compliance but also helps to prevent exposure of leaked aerosol to the eyes and other portions of the face. Efficacy may be increased over existing facemasks that provide little to no incentive to keep the mask in place. Waste may be reduced because the drug is inhaled more efficiently. The time for treatment may also be reduced; for example, the time for relief of asthmatics may be minimized.

Furthermore, the facemasks according to embodiments of the present invention do not substantially change the morphology of existing facemasks. As such, facemasks according to embodiments of the present invention can be used with virtually any nebulizer and MDI spacer. The facemasks can be used with pediatric patients for virtually any disease that requires aerosol therapy including asthma, cystic fibrosis and pulmonary exacerbations of infectious disease. The facemasks generally improve patient compliance, ease of use (e.g., trainability), efficiency and efficacy.

Facemasks according to embodiments of the invention may also be useful for training adults that have difficulty with coordination, such as adults with disabilities and/or the elderly. The audible and/or visual stimuli can improve compliance, efficiency and efficacy with these patients, as well.

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the novel teachings and advantages of this invention. Accordingly, all such modifications are intended to be included within the scope of this invention as defined in the claims. Therefore, it is to be understood that the foregoing is illustrative of the present invention and is not to be construed as limited to the specific embodiments disclosed, and that modifications to the disclosed embodiments, as well as other embodiments, are intended to be included within the scope of the appended claims. The invention is defined by the following claims, with equivalents of the claims to be included therein.

## Claims

1. A facemask (10, 10', 10"), comprising:
a facemask body (12) having a first end (14) including an opening (18) configured to receive at least a portion of a user's face and a skin contact perimeter (22) extending along a periphery of the opening, the skin contact perimeter configured to sealingly engage with a user's face;
a plurality of electrical contacts (24₁, 24₂, 24₃), each electrical contact disposed at a respective region (26₁, 26₂, 26₃) of the skin contact perimeter, each electrical contact configured to sense when the respective region of the skin contact perimeter is sealingly engaged with a user's face; and
a control circuit (28) configured to:
receive a signal from each electrical contact when the respective region of the skin contact perimeter is sealingly engaged with a user's face,
**characterised in that** the control circuit (28) is disposed on the facemask body, and that the control circuit is configured to:
initiate output of an audible stimulus and/or a visual stimulus in response to receiving a concurrent signal from all of the plurality of electrical contacts; and
stop the audible stimulus and/or visual stimulus when one of the electrical contacts (24₁, 24₂, 24₃) does not sense that the respective region of the skin contact perimeter is sealingly engaged with a user's face.

2. The facemask of claim 1, wherein the facemask body includes a port (20) configured to receive medicament from a medicament delivery device, wherein the opening and the port are in fluid communication such that medicament received in the port can be delivered to the opening.

3. The facemask of claim 2, wherein the port is configured to operatively connect with at least one of a nebulizer (50) and a metered dose inhaler (MDI) spacer (60).

4. The facemask of claim 1, further comprising a speaker (34) disposed on the facemask body, the control circuit configured to control the speaker to output an audible stimulus.

5. The facemask of claim 4, wherein the control circuit is configured to control the speaker to output a children's melody and/or song as the audible stimulus.

6. The facemask of claim 1, further comprising at least one light emitting diode (LED) (36) disposed on the facemask body, the control circuit configured to illuminate the at least one LED as a visual stimulus.

7. The facemask of claim 1, further comprising a power source (30) disposed on the facemask body, wherein each electrical contact is a switch configured to be actuated when the respective region of the skin contact perimeter is sealingly engaged with a user's face, the switches configured to electrically connect the power source and the control circuit such that, when all the switches are concurrently actuated, the power source is electrically connected with the control circuit to initiate output of the audible stimulus and/or the visual stimulus.

8. The facemask of claim 7, comprising at least three switches spaced-apart along the skin contact perimeter.

9. The facemask of claim 8, wherein the switches are positioned such that, when the facemask is sealingly engaged with a user's face, one of the switches is actuated by the bridge of the user's nose and two of the switches actuated by the user's face on opposite sides of the user's mouth.

10. The facemask of claim 1, wherein each electrical contact is a capacitive sensor configured to detect contact with skin.

11. The facemask of claim 1, wherein the facemask includes a timer (82) configured to measure an amount of time that a concurrent signal is received from all the plurality of electrical contacts and/or to initiate output of an alarm signal when the measured amount of time reaches a threshold value and/or does not reach a threshold value.

12. The facemask of claim 11, wherein the facemask includes a memory (86) configured to store data received from the timer.

13. The facemask of claim 1, further comprising a transmitter (70) disposed on the facemask body, the transmitter configured to transmit a wireless signal to an outside device (92) that is configured to output the audible stimulus and/or the visual stimulus.

14. The facemask of claim 1, further comprising a transmitter (70) disposed on the facemask body, wherein the transmitter is configured to transmit a wireless signal to an outside device (92) when a concurrent signal is received from all the plurality of electrical contacts such that the outside device can display and/or record data based on the wireless signal.

## Patentansprüche

1. Gesichtsmaske (10, 10', 10"), die Folgendes umfasst:
einen Gesichtsmaskenkörper (12) mit einem ersten Ende (14) mit einer Öffnung (18), die so konfiguriert ist, dass sie zumindest einen Teil des Gesichts eines Benutzers aufnimmt, und einem Hautkontaktprofil (22), das an einer Peripherie der Öffnung entlang verläuft, wobei das Hautkontaktprofil so konfiguriert ist, dass es dicht am Gesicht eines Benutzers anliegt,
mehrere elektrische Kontakte (24₁, 24₂, 24₃), wobei jeder elektrische Kontakt in einem jeweiligen Bereich (26₁, 26₂, 26₃) des Hautkontaktprofils angeordnet ist, wobei jeder elektrische Kontakt so konfiguriert ist, dass er erkennt, ob der jeweilige Bereich des Hautkontaktprofils dicht am Gesicht eines Benutzers anliegt,
einen Steuerkreis (28), der so konfiguriert ist, dass er:
ein Signal von jedem elektrischen Kontakt empfängt, wenn der jeweilige Bereich des Hautkontaktprofils dicht am Gesicht eines Benutzers anliegt,
**dadurch gekennzeichnet, dass** der Steuerkreis (28) an dem Gesichtsmaskenkörper angeordnet und so konfiguriert ist, dass er:
als Reaktion auf das Empfangen eines gleichzeitigen Signals von allen der mehreren elektrischen Kontakte das Ausgeben eines akustischen und/oder eines optischen Reizes veranlasst und
den akustischen und/oder den optischen Reiz einstellt, wenn einer der elektrischen Kontakte (24₁, 24₂, 24₃) nicht erkennt, dass der jeweilige Bereich des Hautkontaktprofils dicht am Gesicht eines Benutzers anliegt.

2. Gesichtsmaske nach Anspruch 1, wobei der Gesichtsmaskenkörper einen Anschluss (20) aufweist, der so konfiguriert ist, dass er aus einer Medikamentabgabevorrichtung ein Medikament aufnimmt, wobei die Öffnung und der Anschluss in Fluidverbindung stehen, so dass im Anschluss aufgenommenes Medikament an die Öffnung abgegeben werden kann.

3. Gesichtsmaske nach Anspruch 2, wobei der Anschluss so konfiguriert ist, dass er sich mit einem Vernebler (50) oder/und einem Abstandshalter (60) für Dosieraerosole (MDI - Metered Dose Inhaler) wirkverbinden lässt.

4. Gesichtsmaske nach Anspruch 1, die ferner einen Lautsprecher (34) umfasst, der an dem Gesichtsmaskenkörper angeordnet ist, wobei der Steuerkreis so konfiguriert ist, dass er den Lautsprecher so steuert, dass dieser einen akustischen Reiz ausgibt.

5. Gesichtsmaske nach Anspruch 4, wobei der Steuerkreis so konfiguriert ist, dass er den Lautsprecher so steuert, dass dieser eine Kindermelodie und/oder ein Lied als akustischen Reiz ausgibt.

6. Gesichtsmaske nach Anspruch 1, die ferner mindestens eine Leuchtdiode (LED) (36) umfasst, welche an dem Gesichtsmaskenkörper angeordnet ist, wobei der Steuerkreis so konfiguriert ist, dass er als optischen Reiz die mindestens eine LED aufleuchten lässt.

7. Gesichtsmaske nach Anspruch 1, die ferner eine Stromquelle (30) umfasst, welche an dem Gesichtsmaskenkörper angeordnet ist, wobei es sich bei jedem elektrischen Kontakt um einen Schalter handelt, der so konfiguriert ist, dass er betätigt wird, wenn der jeweilige Bereich des Hautkontaktprofils dicht am Gesicht eines Benutzers anliegt, wobei die Schalter so konfiguriert sind, dass sie die Stromquelle und den Steuerkreis elektrisch verbinden, so dass die Stromquelle, wenn alle Schalter gleichzeitig betätigt werden, elektrisch mit dem Steuerkreis verbunden und so das Ausgeben des akustischen und/oder des optischen Reizes veranlasst wird.

8. Gesichtsmaske nach Anspruch 7, die mindestens drei Schalter umfasst, welche an dem Hautkontaktprofil entlang voneinander beabstandet sind.

9. Gesichtsmaske nach Anspruch 8, wobei die Schalter so positioniert sind, dass einer der Schalter, wenn die Gesichtsmaske dicht am Gesicht eines Benutzers anliegt, durch den Nasenrücken des Benutzers betätigt wird und zwei der Schalter auf gegenüberliegenden Seiten des Mundes des Benutzers durch das Gesicht des Benutzers betätigt werden.

10. Gesichtsmaske nach Anspruch 1, wobei es sich bei jedem elektrischen Kontakt um einen kapazitiven Sensor handelt, der so konfiguriert ist, dass er einen Kontakt mit der Haut erkennt.

11. Gesichtsmaske nach Anspruch 1, wobei die Gesichtsmaske einen Zeitgeber (82) aufweist, der so konfiguriert ist, dass er eine Zeitspanne misst, in der von allen der mehreren elektrischen Kontakte ein gleichzeitiges Signal eingeht, und/oder das Ausgeben eines Alarmsignals veranlasst, wenn die gemessene Zeitspanne einen Schwellwert erreicht und/oder nicht erreicht.

12. Gesichtsmaske nach Anspruch 11, wobei die Gesichtsmaske einen Speicher (86) aufweist, der so konfiguriert ist, dass er vom Zeitgeber eingehende Daten speichert.

13. Gesichtsmaske nach Anspruch 1, die ferner einen Sender (70) umfasst, der an dem Gesichtsmaskenkörper angeordnet ist, wobei der Sender so konfiguriert ist, dass er ein Funksignal zu einer externen Vorrichtung (92) sendet, die so konfiguriert ist, dass sie den akustischen und/oder den optischen Reiz ausgibt.

14. Gesichtsmaske nach Anspruch 1, die ferner einen Sender (70) umfasst, der an dem Gesichtsmaskenkörper angeordnet ist, wobei der Sender so konfiguriert ist, dass er ein Funksignal zu einer externen Vorrichtung (92) sendet, wenn von allen der mehreren elektrischen Kontakte ein gleichzeitiges Signal eingeht, so dass die externe Vorrichtung auf der Grundlage des Funksignals Daten anzeigen und/oder aufzeichnen kann.

## Revendications

1. Masque de protection (10, 10', 10"), comprenant :
un corps de masque de protection (12) présentant une première extrémité (14) comportant une ouverture (18) configurée pour recevoir au moins une partie du visage d'un utilisateur et un périmètre de contact avec la peau (22) s'étendant le long d'une périphérie de l'ouverture, le périmètre de contact avec la peau étant configuré pour faire contact de manière hermétique avec le visage d'un utilisateur ;
une pluralité de contacts électriques (24₁, 24₂, 24₃), chaque contact électrique étant disposé au niveau d'une région respective (26₁, 26₂, 26₃) du périmètre de contact avec la peau, chaque contact électrique étant configuré pour détecter quand la région respective du périmètre de contact avec la peau est en contact hermétique avec le visage d'un utilisateur ; et
un circuit de commande (28) configuré pour :
recevoir un signal depuis chaque contact électrique quand la région respective du périmètre de contact avec la peau est en contact hermétique avec le visage d'un utilisateur,
**caractérisé en ce que** le circuit de commande (28) est disposé sur le corps de masque de protection, et **en ce que** le circuit de commande est configuré pour :
lancer la sortie d'un stimulus audible et/ou d'un stimulus visuel en réponse à la réception d'un signal simultané depuis la totalité de la pluralité de contacts électriques ; et
arrêter le stimulus audible et/ou le stimulus visuel quand l'un des contacts électriques (24₁, 24₂, 24₃) ne détecte pas que la région respective du périmètre de contact avec la peau est en contact hermétique avec le visage d'un utilisateur.

2. Masque de protection selon la revendication 1, dans lequel le corps de masque de protection comporte un port (20) configuré pour recevoir un médicament depuis un dispositif de délivrance de médicament, dans lequel l'ouverture et le port sont en communication fluidique de telle sorte que le médicament reçu dans le port puisse être délivré à l'ouverture.

3. Masque de protection selon la revendication 2, dans lequel le port est configuré pour se connecter fonctionnellement à au moins l'un d'un nébuliseur (50) et d'un tube d'espacement d'aérosol-doseur (MDI) (60).

4. Masque de protection selon la revendication 1, comprenant en outre un haut-parleur (34) disposé sur le corps de masque de protection, le circuit de commande étant configuré pour commander le haut-parleur afin de produire en sortie un stimulus audible.

5. Masque de protection selon la revendication 4, dans lequel le circuit de commande est configuré pour commander le haut-parleur afin de produire en sortie une mélodie et/ou chanson enfantine comme stimulus audible.

6. Masque de protection selon la revendication 1, comprenant en outre au moins l'une d'une diode électroluminescente (DEL) (36) disposée sur le corps de masque de protection, le circuit de commande étant configuré pour allumer l'au moins une DEL comme stimulus visuel.

7. Masque de protection selon la revendication 1, comprenant en outre une source de puissance (30) disposée sur le corps de masque de protection, dans lequel chaque contact électrique est un commutateur configuré pour être actionné quand la région respective du périmètre de contact avec la peau est en contact hermétique avec le visage d'un utilisateur, les commutateurs étant configurés pour connecter électriquement la source de puissance et le circuit de commande de telle sorte que, quand tous les commutateurs sont actionnés simultanément, la source de puissance soit connectée électriquement au circuit de commande pour lancer la sortie du stimulus audible et/ou du stimulus visuel.

8. Masque de protection selon la revendication 7, comprenant au moins trois commutateurs espacés le long du périmètre de contact avec la peau.

9. Masque de protection selon la revendication 8, dans lequel les commutateurs sont positionnés de telle sorte que, quand le masque de protection est en contact hermétique avec le visage d'un utilisateur, l'un des commutateurs soit actionné par le pont du nez de l'utilisateur et deux des commutateurs soient actionnés par le visage de l'utilisateur sur des côtés opposés de la bouche de l'utilisateur.

10. Masque de protection selon la revendication 1, dans lequel chaque contact électrique est un capteur capacitif configuré pour détecter un contact avec la peau.

11. Masque de protection selon la revendication 1, dans lequel le masque de protection comporte une minuterie (82) configurée pour mesurer une durée de temps pendant laquelle un signal simultané est reçu depuis la totalité de la pluralité de contacts électriques et/ou lancer la sortie d'un signal d'alarme quand la durée de temps mesurée atteint une valeur limite et/ou n'atteint pas une valeur limite.

12. Masque de protection selon la revendication 11, dans lequel le masque de protection comporte une mémoire (86) configurée pour mémoriser des données reçues depuis la minuterie.

13. Masque de protection selon la revendication 1, comprenant en outre un émetteur (70) disposé sur le corps de masque de protection, l'émetteur étant configuré pour transmettre un signal en mode sans fil à un dispositif extérieur (92) qui est configuré pour produire en sortie le stimulus audible et/ou le stimulus visuel.

14. Masque de protection selon la revendication 1, comprenant en outre un émetteur (70) disposé sur le corps de masque de protection, l'émetteur étant configuré pour transmettre un signal en mode sans fil à un dispositif extérieur (92) quand un signal simultané est reçu depuis la totalité de la pluralité de contacts électriques de telle sorte que le dispositif extérieur puisse afficher et/ou enregistrer des données en fonction du signal transmis en mode sans fil.
